# DEMANDE DE BREVET EUROPEEN

(11) **EP 4 450 050 A1**
(43) Date de publication de la demande: **23.10.2024**
(21) Numéro de dépôt: 23305592.0
(22) Date de dépôt: 18.04.2023
(51) Int. Cl.: A61K 8/365, A61K 8/55, A61K 8/73, A61Q 1/02, A61Q 19/00, A61K 8/9728

(54) **COMPOSITION À BASE DE CHITOSAN ET D'UN PHOSPHOLIPIDE**

(71) Demandeur: L'OREAL, 75008 Paris (FR)
(72) Inventeur: LE VERGE, Danielle, 94550 CHEVILLY LARUE (FR); DAVID, Bernadette, 94550 CHEVILLY LARUE (FR); KUSINA, Christophe, 94550 CHEVILLY LARUE (FR)
(74) Mandataire: Lavoix

(57) **Abrégé**

La présente invention concerne une composition cosmétique comprenant, dans un milieu aqueux physiologiquement acceptable :
a) au moins 0,01 % en poids par rapport au poids total de composition de chitosan natif ayant un poids moléculaire strictement supérieur à 3000 Daltons, cette quantité étant strictement inférieure à 15% en poids, et
b) au moins un phospholipide.

## Description

La présente invention concerne une composition cosmétique comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids, et
b) au moins un phospholipide.

Elle se rapporte également à un procédé de maquillage et/ou de soin de la peau et/ou des phanères, dans lequel on applique la composition selon l'invention sur la peau et/ou les phanères.

Il existe de nombreuses compositions cosmétiques pour lesquelles des propriétés de tenue du film déposé, après application sur les matières kératiniques, sont souhaitées. On peut citer par exemple les rouges à lèvres ou les vernis à ongles. Afin d'obtenir un tel résultat, il est possible d'associer des matières premières particulières, notamment des agents filmogènes. Cependant, la présence de tels agents pour la tenue du film sur les matières kératiniques peut conduire à des compositions desséchantes. Par ailleurs, les agents filmogènes couramment utilisés sont généralement de nature pétrochimique, comme les silicones ou les polymères acryliques.

Par ailleurs, on cherche souvent à obtenir des compositions couvrantes.

Les systèmes à base de chitosan montrent que l'association de chitosan et d'un pigment permet de générer des films résistants à la friction à sec et à l'huile. Néanmoins, la sensibilité à l'eau de ces dépôts est très forte et reste un challenge majeur des technologies à base de chitosan.

Le formulateur est donc à la recherche de matières premières et/ou de systèmes permettant d'obtenir des compositions fluides à base de chitosan (i.e. qui s'écoulent) dont le dépôt est couvrant, homogène, présentant une bonne tenue et dont la résistance à l'eau est améliorée.

Par ailleurs, la formulation de produits cosmétiques respectueux de l'environnement, c'est-à-dire dont la conception et le développement tiennent compte des enjeux environnementaux, devient une préoccupation majeure pour contribuer à relever les défis planétaires.

Il se révèle donc essentiel de proposer des compositions et/ou des procédés de préparation et/ou des ingrédients plus durables permettant ainsi de répondre à ces enjeux environnementaux.

Dans ce contexte, il est important de développer de nouvelles compositions cosmétiques avec une meilleure empreinte carbone notamment en favorisant l'emploi de matières premières renouvelables et/ou avec un bon index de naturalité et/ou d'origine naturelle et plus particulièrement d'origine végétale tout en réduisant l'utilisation de composés d'origine pétrochimique.

La présente invention a pour but de proposer des compositions cosmétiques aqueuses, colorées ou non, présentant de bonnes propriétés, notamment en termes d'homogénéité et de bonne tenue, en particulier ayant une bonne tenue/résistance à l'eau.

Après application, ces compositions laissent un dépôt filmogène couvrant et homogène, qui a une bonne tenue à l'usure. Les films colorés formés sont adhésifs et cohésifs, et présentent une résistance améliorée à l'eau.

Ces compositions comprennent également des ingrédients durables, permettant ainsi de répondre aux enjeux environnementaux.

La présente invention a donc pour objet une composition cosmétique, notamment de maquillage et/ou de soin de la peau et/ou des lèvres, en particulier des lèvres, comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids, et
b) au moins un phospholipide.

Par « physiologiquement acceptable », on entend un milieu compatible avec les matières kératiniques.

Elle a également pour objet un procédé de maquillage et/ou de soin de la peau et/ou des phanères, dans lequel on applique la composition selon l'invention sur la peau et/ou les phanères.

Elle a également pour objet un procédé de maquillage et/ou de soin de la peau et/ou des phanères, dans lequel on applique la composition selon l'invention sur la peau et/ou les phanères.

De préférence, la composition selon l'invention est substantiellement exempte de polymère (méth)acrylique. De préférence, la composition selon l'invention est substantiellement exempte de silicone.

Par « substantiellement exempte de polymère (méth)acrylique », on entend que la composition comprend moins de 1% en poids par rapport au poids total de la composition, de préférence moins de 0,5% en poids, de préférence moins de 0,3% en poids, préférentiellement 0,1% en poids de polymère (méth)acrylique. De préférence, la composition est totalement exempte de polymère (méth)acrylique. Par polymère (méth)acrylique, on entend un polymère comprenant au moins un monomère d'acide acrylique ou au moins un monomère d'acide méthacrylique.

Par « substantiellement exempte de silicone », on entend que la composition comprend moins de 1 % en poids par rapport au poids total de la composition, de préférence moins de 0,5% en poids, de préférence moins de 0,3% en poids, préférentiellement 0,1% en poids de silicone. De préférence, la composition est totalement exempte de silicone. Par silicone, on entend tout composé siliconé.

### Chitosan

La composition selon l'invention comprend au moins 0,01% en poids par rapport au poids total de la composition d'au moins un chitosan natif ayant un poids moléculaire strictement supérieur à 3000 Daltons (3 kDa).

La quantité de chitosan natif est également strictement inférieure à 15% en poids par rapport au poids total de la composition.

De préférence, le chitosan natif a un poids moléculaire supérieur ou égal à 10 kDa, de préférence supérieur ou égal à 15 kDa, de préférence supérieur ou égal à 20 kDa. De préférence, le chitosan natif a un poids moléculaire compris entre 10 kDa et 2 MDa, de préférence compris entre 15 kDa et 1,5 MDa, de préférence compris entre 20 kDa et 300 kDa, de préférence compris entre 20 kDa et 200 kDa.

Le chitosan (ou chitosane) est très peu répandu dans la nature. Il n'est signalé que dans les exosquelettes de certains insectes comme les reines des termites et dans les parois cellulaires d'une classe particulière de champignons, les zygomycètes.

Le chitosan est obtenu par désacétylation de la chitine. La chitine est un polysaccharide composé de plusieurs unités N-acétyl-D-glucosamine reliées entre elles par une liaison de type β (1,4).

La structure chimique idéale du chitosane est un enchaînement de monomères β-D-glucosamine reliés par une liaison glycosidique (1→4).

Par « chitosan » selon l'invention, on entend tout copolymère formé d'unités constitutives N-acétyl-D-glucosamine et D-glucosamine, dont le degré d'acétylation est inférieur à 90%. Le chitosan est constitué des unités sucres glucosamine (unités désacétylées) et d'unités N-acétyl-D-glucosamine (unités acétylées) reliées entre elles par des liaisons de type β (1,4) et constitue un polymère du type Poly(N-acetyl-D-glucosamine) -poly(D-glucosamine).

De préférence, le degré d'acétylation du chitosan est inférieur ou égal à 80%, de préférence inférieur ou égal à 70%, de préférence inférieur ou égal à 60%, de préférence inférieur ou égal à 50%, de préférence inférieur ou égal à 35%, de préférence inférieur ou égal à 25%, de préférence inférieur ou égal à 15%.

Le degré d'acétylation est le pourcentage d'unités acétylées par rapport au nombre d'unités totales, il peut être déterminé par spectroscopie infrarouge à transformée de Fourier (IR-TF) ou par un titrage par une base forte.

Le chitosan de l'invention est de préférence un polysaccharide préparé à partir d'une origine fongique. Il est notamment extrait et purifié à partir de sources fongiques alimentaires ou biotechnologique sûres et abondantes tels que *Agaricus bisporus* ou *Aspergillus niger.*

Le chitosan de l'invention est de préférence issu du mycélium d'un champignon du type Ascomycète, et en particulier *d'Aspergillus niger* et/ou d'un champignon Basidiomycète, et en particulier *Lentinula edodes* (shiitake) et/ou *Agaricus bisporus.* De préférence le champignon est *Aspergillus niger.*

Le chitosan peut être d'origine OGM, mais de préférence est d'origine non OGM.

Le chitosan selon l'invention est natif, c'est-à-dire qu'il n'est pas modifié. Il ne contient notamment pas de modification chimique.

Une méthode de préparation du chitosan est celle décrite dans la demande WO03068824.

De préférence, le chitosan utilisé dans l'invention est sous forme de poudre. Il est notamment commercialisé par Kitozyme sous le nom Kiosmetine ou Kionutrime.

Le chitosan est de préférence présent en une quantité allant de 0,01% à 14% en poids, de préférence de 0,1% à 14% en poids, de préférence de 0,1% à 12% en poids, de préférence de 0,2% à 7% en poids, de préférence de 0,25% à 5% en poids par rapport au poids total de la composition.

### Phospholipide

La composition selon l'invention comprend au moins un phospholipide.

Le phospholipide est de préférence choisi parmi :
- les phospholipides naturels, notamment la lécithine d'oeuf ou de soja, ou la sphingomyéline,
- les phospholipides modifiés par voie chimique ou enzymatique, notamment la lécithine hydrogénée, et
- les phospholipides de synthèse, notamment la dipalmitoylphosphatidylcholine (DPPC).

Le phospholipide est de préférence choisi parmi les phospholipides naturels et les phospholipides modifiés par voie chimique ou enzymatique.

Le phospholipide est de préférence choisi parmi la lécithine commercialisée sous la dénomination EMULMETIK 100J et la lécithine hydrogénée commercialisée sous la dénomination NIKKOL LECINOL S 10. De préférence, le phospholipide est la lécithine hydrogénée.

De préférence, le phospholipide est présent dans un mélange avec des alcools gras comme des alcools ayant de 12 à 16 atomes de carbone, et/ou avec un acide gras tel que l'acide palmitique. Préférentiellement, le phospholipide est commercialisé sous le nom INCI C12-16 ALCOHOLS (and) PALMITIC ACID (and) HYDROGENATED LECITHIN, et notamment sous la référence NIKKOL LECINOL S 10.

Le phospholipide est présent dans la composition selon l'invention en quantité de préférence allant de 0,1% à 10% en poids par rapport au poids total de la composition, de préférence de 0,1% à 5% en poids, de préférence de 0,1% à 3% en poids, de préférence de 0,45% à 2%, mieux de 0,75% à 1,5%, par rapport au poids total de la composition.

Selon l'invention, le ratio pondéral entre le chitosan et le phospholipide (i.e. ratio pondéral chitosan : phospholipide) est compris entre 0,1 et 10. De préférence, il est compris entre 0,2 et 10, de préférence compris entre 0,3 et 5, de préférence entre 0,5 et 3.

### Matières colorantes pigmentaires

La composition selon l'invention peut comprendre en outre au moins une matière colorante pigmentaire. Cette matière colorante est choisie parmi les matières colorantes pulvérulentes comme les pigments minéraux, les nacres, les pigments organiques.

On entend par « pigments » des particules blanches ou colorées, minérales ou organiques, insolubles dans un milieu aqueux, destinées à colorer la composition et/ou le dépôt résultant.

Les matières colorantes peuvent être présentes, dans la composition, en une teneur allant de 1% à 70% en poids, de préférence de 1% à 60% en poids, de préférence de 1% à 50% en poids, de préférence de 3% à 45% en poids, par rapport au poids de la composition, de préférence de 4% à 30% en poids, de préférence de 5% à 20% en poids, de préférence de 6% à 15% en poids.

### Pigments minéraux

Selon un mode de réalisation particulier, les pigments utilisés selon l'invention sont choisis parmi les pigments minéraux.

Par « pigment minéral », on entend tout pigment qui répond à la définition de l'encyclopédie Ullmann dans le chapitre pigment inorganique. On peut citer, parmi les pigments minéraux utiles dans la présente invention, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, le dioxyde de titane, les poudres métalliques comme la poudre d'aluminium et la poudre de cuivre. Les pigments minéraux suivants peuvent aussi être utilisés : Ta2O5, Ti3O5, Ti2O3, TiO, ZrO2 en mélange avec TiO2, ZrO2, Nb2O5, CeO2, ZnS.

La taille du pigment utile dans le cadre de la présente invention est en général supérieure à 100 nm et peut aller jusqu'à 10 µm, de préférence de 200 nm à 5 µm, et plus préférentiellement de 300 nm à 1 µm. Selon une forme particulière de l'invention, les pigments présentent une taille caractérisée par un D[50] supérieur à 100 nm et pouvant aller jusqu'à 10 µm, de préférence de 200 nm à 5µm, et plus préférentiellement de 300 nm à 1 µm. Les tailles sont mesurées par diffusion statique de la lumière au moyen d'un granulomètre commercial de type MasterSizer 3000^{®} de chez Malvern, permettant d'appréhender la répartition granulométrique de l'ensemble des particules sur une large gamme pouvant aller de 0,01 µm à 1000 µm. Les données sont traitées sur la base de la théorie classique de diffusion de Mie. Cette théorie est la plus adaptée pour des distributions de taille allant du submicronique au multi-micronique, elle permet de déterminer un diamètre « effectif » de particules. Cette théorie est notamment décrite dans l'ouvrage de Van de Hulst, H.C., « Light Scattering by Small Particles », Chapitres 9 et 10, Wiley, New York, 1957. D[50] représente la taille maximale que présente 50 % en volume les particules.

Dans le cadre de la présente invention, les pigments minéraux sont plus particulièrement l'oxyde de fer et/ou le dioxyde de titane.

Comme pigments minéraux utilisables dans l'invention, on peut également citer les nacres. Par « nacres », il faut comprendre des particules colorées de toute forme, irisées ou non, notamment, produites par certains mollusques dans leur coquille ou bien synthétisées et qui présentent un effet de couleur par interférence optique.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques. On peut également citer, à titre d'exemple de nacres, le mica naturel recouvert d'oxyde de titane, d'oxyde de fer, de pigment naturel ou d'oxychlorure de bismuth. Les nacres peuvent plus particulièrement posséder une couleur ou un reflet jaune, rose, rouge, bronze, orangé, brun, or et/ou cuivré.

Parmi les pigments utilisables selon l'invention, on peut également citer ceux à effet optique différent d'un simple effet de teinte conventionnel, c'est-à-dire unifié et stabilisé tel que produit par les matières colorantes classiques, comme par exemple, les pigments monochromatiques.

Au sens de l'invention, « stabilisé » signifie dénué d'effet de variabilité de la couleur avec l'angle d'observation ou encore en réponse à un changement de température. Par exemple, ce matériau peut être choisi parmi les particules à reflet métallique, les agents de coloration goniochromatiques, les pigments diffractants, les agents thermochromes, les agents azurants optiques, ainsi que les fibres, notamment, interférentielles. Bien entendu, ces différents matériaux peuvent être associés de manière à procurer la manifestation simultanée de deux effets, voire d'un nouvel effet conforme à l'invention.

Selon un mode particulier, la composition selon l'invention comprend au moins un pigment non enrobé.

### Pigments organiques

Selon un autre mode de réalisation de l'invention, la matière colorante pigmentaire est un pigment organique, synthétique, naturel ou d'origine naturelle.

Par « pigment organique », on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

Le ou les pigments organiques peuvent être choisis par exemple parmi le carmin, le noir de carbone, le noir d'aniline, la mélanine, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les références CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, et les pigments obtenus par polymérisation oxydante de dérivés indoliques, phénoliques tels qu'ils sont décrits dans le brevet FR 2 679 771.

Les pigments peuvent aussi être sous forme de pigments composites tels qu'ils sont décrits dans le brevet EP 1 184 426. Ces pigments composites peuvent être composés notamment de particules comportant un noyau inorganique recouvert au moins partiellement d'un pigment organique et au moins un liant assurant la fixation des pigments organiques sur le noyau.

Le pigment peut aussi être une laque.

Par laque, on entend les colorants insolubilisés adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants organiques, on peut citer le carmin de cochenille. On peut également citer les produits connus sous les dénominations suivantes : D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 1 O (CI 77 002), D&C Green 3 (CI 42 053), D&C Blue 1 (CI 42 090). A titre d'exemples de laques, on peut citer le produit connu sous la dénomination D&C Red 7 (CI 15 850 :1).

### Charges

La composition selon l'invention peut comprendre également au moins une charge.

Par « charges », il faut comprendre des particules de toute forme, incolores ou blanches, minérales ou de synthèses, insolubles et dispersées dans le milieu de la composition quelle que soit la température à laquelle la composition est fabriquée. De manière générale, les charges comprises dans les compositions selon l'invention ne sont pas des matières colorantes pigmentaires.

De préférence, la charge est choisie parmi les particules de cellulose, les silices, l'amidon, le kaolin, les argiles, les particules de nylon ou de polyméthacrylate de méthyle (PMMA) et leurs mélanges.

Les particules de cellulose utilisables selon l'invention sont de préférence sphériques (billes de cellulose).

Par particules sphériques au sens de la présente invention, on entend des particules pleines ou poreuses ayant un paramètre de circularité d'au moins 0,95. Le paramètre de circularité est défini comme le rapport de la circonférence d'un disque ayant la même aire que la particule au périmètre de la particule. Une valeur de 1 caractérise des particules parfaitement sphériques.

Elles présentent de préférence une taille moyenne inférieure à 40 µm, de préférence allant de 1 à 20 µm, encore préférentiellement de 2 à 10 µm.

Parmi les particules de cellulose utilisables selon l'invention, on peut citer en particulier celles vendues par la société Daito sous la marque CELLULOBEADS^{®} telles que CELLULOBEADS USF^{®} (D[50] = 4 µm), CELLULOBEADS D-5^{®} (D[50] < 10 µm), CELLULOBEADS D-10^{®} (D[50] < 15 µm), CELLULOBEADS D-30^{®} (D[50] < 30 µm).

De préférence, les charges sont présentes dans une teneur allant de 0,1% à 20% en poids par rapport au poids total de la composition, de préférence de 0,2% à 15% en poids, par rapport au poids de la composition, de préférence de 0,3% à 10% en poids, de préférence de 0,4% à 5% en poids, de préférence de 0,5% à 4% en poids.

### Alpha hydroxy acide (AHA)

La composition selon l'invention peut comprendre au moins un AHA.

Par alpha hydroxy acide, on entend selon la présente invention un acide carboxylique ayant au moins une fonction hydroxy occupant une position alpha sur ledit acide (carbone adjacent à une fonction acide carboxylique). Cet acide peut se présenter dans la composition finale sous forme d'acide libre et/ou sous la forme de l'un de ses sels associés (sels avec une base organique ou un alcalin notamment), en particulier selon le pH final imposé à la composition.

Les α-hydroxyacides (alpha hydroxyacides ou AHA) sont par exemple choisis parmi l'acide lactique, l'acide citrique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxy-hexadécanoïque, l'acide 2-hydroxyoctadécanoïque, l'acide 2-hydroxytétra-cosanoïque, l'acide 2-hydroxyeïcosanoïque ; l'acide mandélique ; l'acide phényllactique ; l'acide gluconique ; l'acide galacturonique ; l'acide aleuritique ; l'acide ribonique ; l'acide tartronique ; l'acide tartrique ; l'acide malique ; l'acide fumarique ; leurs sels et leurs mélanges.

Selon un mode préféré, l'alpha hydroxyacide est choisi parmi l'acide lactique, l'acide citrique, l'acide malique, l'acide tartrique et leurs sels. Plus particulièrement, l'alpha hydroxyacide est choisi parmi l'acide lactique, l'acide citrique, leurs sels et leurs mélanges.

Le ou les alpha hydroxyacides peuvent être présents en une quantité allant de 0,001 à 10% en poids, de 0,005 à 5% en poids, de préférence de 0,01 à 3% en poids par rapport au poids total de la composition.

### Milieu aqueux physiologiquement acceptable

La composition selon l'invention comprend un milieu aqueux physiologiquement acceptable. Ledit milieu comprend de l'eau.

L'eau utilisée peut être de l'eau déminéralisée stérile et/ou une eau florale telle que de l'eau de rose, de l'eau de bleuet, de l'eau de camomille ou de l'eau de tilleul, et/ou une eau thermale ou minérale naturelle.

La composition comprend de préférence au moins 5% en poids d'eau par rapport au poids total de la composition, de préférence au moins 10% en poids, de préférence au moins 20% en poids, de préférence au moins 30% en poids, de préférence au moins 40% en poids. La composition comprend de préférence de 5% à 95% en poids d'eau par rapport au poids total de la composition, plus préférentiellement de 10% à 85%, encore plus préférentiellement de 20% à 80%, encore plus préférentiellement de 25% à 70%, encore plus préférentiellement de 28% à 60%, encore plus préférentiellement de 30% à 50%.

La phase aqueuse peut également comprendre au moins un solvant organique miscible dans l'eau à 25°C.

De préférence, le solvant organique miscible dans l'eau est choisi parmi les alcools, les polyols et leurs mélanges.

Parmi les alcools, on peut citer les alcools en C₁-C₁₀, plus préférentiellement en C₁-C₅, tels que l'éthanol, l'isopropanol, le propanol et le butanol.

Le polyol est, de préférence, choisi parmi les polyols ayant de 2 à 20 atomes de carbone, plus préférentiellement de 2 à 6 atomes de carbone, comme le glycérol, le diglycérol, le propylèneglycol, l'isoprène glycol, le dipropylèneglycol, le butylène glycol, l'hexylène glycol, le 1,2-propanediol, le 1,3-propanediol, le pentylène glycol, les polyéthylèneglycols ayant de 2 à 200 motifs d'oxyde d'éthylène et leurs mélanges.

La composition peut comprendre de 1% à 25% en poids de solvant organique miscible dans l'eau, par rapport au poids total de la composition, plus préférentiellement de 2% à 20% en poids, encore plus préférentiellement de 3% à 15% en poids.

### pH de la composition

La composition selon l'invention présente un pH inférieur ou égal à 7, de préférence inférieur ou égal à 6,5, de préférence inférieur ou égal à 6,3. Avantageusement, le pH de la composition est compris entre 3 et 6,3, de préférence compris entre 4 et 6,3.

De préférence, la composition cosmétique selon l'invention comprend au moins une base et/ou au moins un acide. La base et l'acide selon l'invention sont connus et classiquement utilisés dans le domaine cosmétique.

La base et/ou l'acide sont notamment utilisés pour ajuster le pH final de la composition entre 3 et 6,3.

L'acide peut par exemple être l'acide citrique.

La base peut être choisie parmi les bases minérales comme par exemple les hydroxydes de métaux alcalins, l'hydroxyde de sodium, l'hydroxyde de potassium.

De préférence, la base de la composition est un hydroxyde de métal alcalin, de préférence l'hydroxyde de sodium ou l'hydroxyde de potassium.

La composition selon l'invention peut comprendre au moins une base en une teneur en matière active allant de 0,5 % à 10 % en poids, par rapport au poids total de la composition, notamment de 1 % à 5 % en poids, de préférence allant de 1 % à 4 % en poids.

La composition conforme à l'invention peut être obtenue de manière classique par l'homme du métier.

Les exemples qui suivent permettront de mieux comprendre l'invention, sans toutefois présenter un caractère limitatif. Les matières premières sont nommées par leur nom chimique ou INCI. Les quantités indiquées sont en % en poids de matières premières par rapport au poids total de composition (% p/p), sauf mention contraire.

### Exemples: préparation de compositions selon l'invention et comparaisons avec une composition comparative

La composition B, C et D selon l'invention, et A comparative, ont été préparées par mélange des ingrédients du tableau 1.

La composition B, C et D selon l'invention contiennent de la lécithine hydrogénée et des pigments.

La composition A comparative contient du chitosan et des pigments mais pas de lécithine hydrogénée.

### - Protocole d'étalement des compositions en un film

L'étalement des produits se fait sur un banc d'étalement (Elcometer 4340 Applicator) permettant de régler sa vitesse ainsi que la distance sur lequel il se fait. Le banc est équipé d'un système d'aspiration relié à une pompe pour que le support où l'on fait l'étalement, ne bouge pas. Des films transparents sont utilisées (byko-chart, code 2871). L'épaisseur d'étalement est quant à elle réglable grâce à l'étaleur carré déposé sur le support de manière à étaler par arasement lorsque la plateforme est mise en marche. Chaque tranche de l'étaleur permet d'étaler avec une épaisseur différente allant de 25µm à 200µm. L'épaisseur choisie est de 25 µm afin de se rapprocher d'une épaisseur du film *in vivo.* Un poids de 960 g est ajouté par-dessus l'étaleur pendant l'étalement. La vitesse de l'étalement est réglée à 1in/sec, soit 2,54cm/s. Les films sont séchés pendant 24h à température et humidité (HR) ambiante (50% HR).

### - Protocole de test de tenue aux frottements à l'eau

Le test de tenue aux frottements à l'eau est réalisé par les mesures colorimétriques sur film sec avant et après abrasion. L'abrasion est effectuée en fixant une bandelette de mouchoir en tissu (Chicopee Veraclean Polish Plus) mouillé (400 µL d'eau dans le mouchoir en tissu) sur la tranche d'étaleur à 25 µm. Le poids de 960 g est ajouté par-dessus l'étaleur pendant l'abrasion. La vitesse de banc est réglée à 2,54 cm/s.

La mesure de la couleur avant est après abrasion est faite avec la même méthode spectrophotométrique qu'expliquée précédemment.

Afin d'évaluer la tenue aux frottements, le « Contraste Ratio » respectivement avant le frottement (CR Dépôt Sec, %) et après l'abrasion (CR Frot Dépôt Sec, %) sont mesurés. La perte [(CR Frot Dépôt Sec - CR Dépôt sec] / CR Dépôt Sec]*100, en pourcentage, quantifie la perte de couvrance et indique la tenue du film aux frottements : plus cette perte est faible, plus le film est résistant aux frottements.

Chaque valeur de CR représente donc une moyenne de 3 à 6 mesures. Les résultats sont dans le tableau 1.

Les résultats sont dans le tableau 1.

**[Table 1]**

| **Ingrédients (% p/p)** | **A comparative** | **B selon l'invention** | **C selon l'invention** | **D selon l'invention** |
|---|---|---|---|---|
| Chitosan (Kiosmetine-CSH de Kitozyme) | 2 | 2 | 2 | 2 |
| Acide lactique | 1 | 1 | 1 | 1 |
| Pigments* | 15 | 15 | 15 | 15 |
| Lécithine hydrogénée (NIKKOL LECINOL S 10) | - | 0,5 | 1 | 1,5 |
| Eau | Qsp 100 | Qsp 100 | Qsp 100 | Qsp 100 |
| **Perte friction eau %(+/-%)** | **-54(13)** | **-27(5)** | **-29(6)** | **-34(5)** |

| | | | | |
|---|---|---|---|---|
| * Les pigments utilisés dans les compositions sont un mélange de dioxyde de titane commercialisé sous la dénomination HOMBITAN FF PHARMA-VENATOR, d'oxyde de fer rouge commercialisé sous la dénomination TAROX IRON OXIDE R-800HP - TITAN KOGYO, d'oxyde de fer jaune commercialisé sous la dénomination TAROX IRON OXIDE BL-100HPL- TITAN KOGYO et d'oxyde de fer noir commercialisé sous la dénomination TAROX IRON OXIDE LL-100HP- TITAN KOGYO. | | | | |

Les résultats montrent une meilleure tenue à l'eau pour les compositions B, C et D selon l'invention, par rapport à la composition comparative A. L'utilisation d'un phopholipide utilisé dans l'invention, apporte ainsi de la résistance à l'eau.

## Revendications

1. Composition cosmétique, notamment de maquillage et/ou de soin de la peau et/ou des lèvres, en particulier des lèvres, comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids, et
b) au moins un phospholipide.

2. Composition selon la revendication 1, dans laquelle le chitosan natif a un poids moléculaire supérieur ou égal à 10 kDa, de préférence supérieur ou égal à 15 kDa, de préférence supérieur ou égal à 20 kDa, de préférence le chitosan natif a un poids moléculaire compris entre 10 kDa et 2 MDa, de préférence compris entre 15 kDa et 1,5 MDa, de préférence compris entre 20 kDa et 300 kDa, de préférence compris entre 20 kDa et 200 kDa.

3. Composition selon la revendication 1 ou 2, dans laquelle le chitosan natif a un degré d'acétylation du chitosan inférieur ou égal à 80%, de préférence inférieur ou égal à 70%, de préférence inférieur ou égal à 60%, de préférence inférieur ou égal à 50%, de préférence inférieur ou égal à 35%, de préférence inférieur ou égal à 25%, de préférence inférieur ou égal à 15%.

4. Composition selon l'une des revendications précédentes, dans laquelle le chitosan natif est un polysaccharide préparé à partir d'une origine fongique, de préférence issu du mycélium d'un champignon du type Ascomycète, et en particulier *d'Aspergillus niger* et/ou d'un champignon Basidiomycète, et en particulier *Lentinula edodes* et/ou *Agaricus bisporus,* de préférence le champignon est *Aspergillus niger.*

5. Composition selon l'une des revendications précédentes, dans laquelle le chitosan natif est présent en une quantité allant de 0,01% à 14% en poids, de préférence de 0,1% à 14% en poids, de préférence de 0,1% à 12% en poids, de préférence de 0,2% à 7% en poids, de préférence de 0,25% à 5% en poids par rapport au poids total de la composition.

6. Composition selon l'une des revendications précédentes, dans laquelle le phospholipide est choisi parmi les phospholipides naturels et les phospholipides modifiés par voie chimique ou enzymatique.

7. Composition selon l'une des revendications précédentes, dans laquelle le phospholipide est choisi parmi la lécithine et la lécithine hydrogénée.

8. Composition selon l'une des revendications précédentes, dans laquelle le phospholipide est présent en une quantité allant de 0,05% à 10% en poids, plus préférentiellement de 0,1% à 5% en poids, et mieux de 0,5% à 2% en poids, par rapport au poids total de la composition.

9. Composition selon l'une des revendications précédentes, dans laquelle le ratio pondéral entre le chitosan et le phospholipide (i.e. ratio pondéral chitosan : phospholipide) est compris entre 0,1 et 10, de préférence compris entre 0,2 et 10, de préférence compris entre 0,3 et 5 et encore plus préférentiellement, compris entre 0,5 et 3.

10. Composition selon l'une des revendications précédentes, comprenant en outre au moins un composé choisi parmi les matières colorantes pigmentaires et les charges.

11. Composition selon la revendication 10, dans laquelle la matière colorante pigmentaire est un pigment minéral choisi parmi le dioxyde de titane, les oxydes de fer, les oxydes de zirconium ou de cérium, les oxydes de zinc ou de chrome, le violet de manganèse, le bleu outremer, l'hydrate de chrome et le bleu ferrique, les poudres métalliques comme la poudre d'aluminium et la poudre de cuivre, les nacres, les pigments monochromatiques ; et/ou la charge est choisie parmi les particules de cellulose, les silices, l'amidon, le kaolin, les argiles, les particules de nylon ou de polyméthacrylate de méthyle et leurs mélanges.

12. Composition selon la revendication 10, dans laquelle la matière colorante pigmentaire est un pigment organique choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

13. Composition selon la revendication 10 ou 11, dans laquelle la matière colorante pigmentaire est choisie parmi les pigments minéraux, de préférence parmi le dioxyde de titane, les oxydes de fer, les oxydes de zirconium ou de cérium, les oxydes de zinc ou de chrome et leurs mélanges, de préférence le pigment est choisi parmi le dioxyde de titane, les oxydes de fer et leurs mélanges.

14. Composition selon l'une des revendications 10 à 14, dans laquelle la matière colorante pigmentaire est présente en une teneur allant de 1% à 70% en poids, de préférence de 1% à 60% en poids, de préférence de 1% à 50% en poids, de préférence de 3% à 45% en poids, par rapport au poids de la composition, de préférence de 4% à 30% en poids, de préférence de 5% à 20% en poids, de préférence de 6% à 15% en poids.

15. Composition selon l'une des revendications précédentes qui est substantiellement exempte de polymère (méth)acrylique et/ou de silicone ; de préférence la composition comprend moins de 1% en poids par rapport au poids total de la composition, de préférence moins de 0,5% en poids, de préférence moins de 0,3% en poids, préférentiellement 0,1% en poids de polymère (méth)acrylique, de préférence la composition est totalement exempte de polymère (méth)acrylique ; de préférence la composition comprend moins de 1% en poids par rapport au poids total de la composition, de préférence moins de 0,5% en poids, de préférence moins de 0,3% en poids, préférentiellement 0,1% en poids de silicone, de préférence la composition est totalement exempte de silicone.

16. Composition selon l'une des revendications précédentes, dans laquelle le milieu aqueux physiologiquement acceptable comprend au moins 5% en poids d'eau par rapport au poids total de la composition, de préférence au moins 10% en poids, de préférence au moins 20% en poids, de préférence au moins 30% en poids, de préférence au moins 40% en poids ; de préférence de 5% à 95% en poids d'eau par rapport au poids total de la composition, plus préférentiellement de 10% à 85%, encore plus préférentiellement de 20% à 80%, encore plus préférentiellement de 25% à 70%, encore plus préférentiellement de 28% à 60%, encore plus préférentiellement de 30% à 50% ; et éventuellement au moins un solvant organique miscible dans l'eau à 25°C choisi parmi les alcools, les polyols et leurs mélanges.

17. Composition selon l'une des revendications précédentes, qui comprend au moins un alpha hydroxy acide, de préférence choisi parmi l'acide lactique, l'acide citrique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxy-hexadécanoïque, l'acide 2-hydroxyoctadécanoïque, l'acide 2-hydroxytétra-cosanoïque, l'acide 2-hydroxyeïcosanoïque ; l'acide mandélique ; l'acide phényllactique ; l'acide gluconique ; l'acide galacturonique ; l'acide aleuritique ; l'acide ribonique ; l'acide tartronique ; l'acide tartrique ; l'acide malique ; l'acide fumarique ; leurs sels et leurs mélanges.

18. Composition selon l'une des revendications précédentes, qui présente un pH inférieur ou égal à 7, de préférence inférieur ou égal à 6,5, de préférence inférieur ou égal à 6,3, de préférence compris entre 3 et 6,3, de préférence compris entre 4 et 6,3.

19. Procédé de maquillage et/ou de soin de la peau et/ou des phanères, dans lequel on applique la composition selon l'une des revendications précédentes sur la peau et/ou les phanères.
